# EUROPEAN PATENT SPECIFICATION

(11) **EP 3 006 019 B1**
(45) Date of publication and mention of the grant of the patent: **31.07.2019**
(21) Application number: 15164516.5
(22) Date of filing: 21.04.2015
(51) Int. Cl.: A61K 9/00, A61K 47/36, A61K 31/00, A61K 38/00

(54) **LONG-LASTING INJECTABLE DRUG RELEASING GEL COMPOSITION AND METHOD OF MANUFACTURING THE SAME**
LANGANHALTENDE INJIZIERBARE WIRKSTOFFFREISETZENDE GELZUSAMMENSETZUNG UND VERFAHREN ZUR HERSTELLUNG DAVON
COMPOSITION À BASE DE GEL À LIBÉRATION PROLONGÉE D'UN MÉDICAMENT INJECTABLE ET PROCÉDÉ DE FABRICATION DE CELLE-CI

(30) Priority: 08.10.2014 TW 103135071
(43) Date of publication of application: 13.04.2016
(73) Proprietor: National Chiao Tung University, Hsinchu City 300 (TW)
(72) Inventor: Liu, Dean-Mo, 302 Jhubei City (TW); Chou, Hao-Syun, 970 Hualien City (TW); Hsiao, Meng-Hsuan, 235 New Taipei City (TW); Chen, Yi-Chieh, 320 Taoyuan City (TW)
(74) Representative: Miller Sturt Kenyon

(56) References cited:
- WO-A2-2013/123492
- V.R SINHA ET AL: "Chitosan microspheres as a potential carrier for drugs", INTERNATIONAL JOURNAL OF PHARMACEUTICS, vol. 274, no. 1-2, 1 April 2004 (2004-04-01), pages 1-33, XP055054504, ISSN: 0378-5173, DOI: 10.1016/j.ijpharm.2003.12.026
- BHATTARAI N ET AL: "Chitosan-based hydrogels for controlled, localized drug delivery", ADVANCED DRUG DELIVERY REVIEWS, ELSEVIER, AMSTERDAM, NL, vol. 62, no. 1, 31 January 2010 (2010-01-31), pages 83-99, XP026877916, ISSN: 0169-409X [retrieved on 2009-09-30]
- MUZZARELLI ET AL: "Genipin-crosslinked chitosan hydrogels as biomedical and pharmaceutical aids", CARBOHYDRATE POLYMERS, APPLIED SCIENCE PUBLISHERS, LTD. BARKING, GB, vol. 77, no. 1, 22 May 2009 (2009-05-22), pages 1-9, XP026019555, ISSN: 0144-8617, DOI: 10.1016/J.CARBPOL.2009.01.016 [retrieved on 2009-01-29]

## Description

### BACKGROUND

### Field of Invention

The present invention relates to a gel composition and method of manufacturing the same. More particularly, the present invention relates to a long-lasting, injectable drug releasing gel composition and method of manufacturing the same.

### Description of Related Art

A lot of patients suffering from chronic diseases need to inject or take medicine at a specific time so as to keep their well-being. Injection medicine is a type of medication that is directly injected into the body. The drug absorption rate is high, while the rate of drug releasing cannot be regulated in this type of treatment. Therefore, the patients have to receive the injection regularly, which can cause certain burden both mentally and physically. Drugs that are orally administered may avoid the inconvenience brought about by the injection, but the majority of drugs cannot be faithfully transported to the target area, such that the absorption rate is low and the efficacy is not prominent.

Take diabetes for example. The common treatment is achieved by regular injection. Patients have to have insulin injection twice a day and monitor their blood sugar value all the time. In addition, the type of injection and dosage may vary according to patient's health condition. It results in tremendous mental and physical burden. In the case of incorrect insulin injection or over/under dosage, it can lead to fatal consequence to the patient. Therefore, there is an urgent need to provide a more reliable and easier treatment.

Among current studies, oral administration of insulin is the most convenient one. However, the insulin absorption rate is relatively low, and it requires a greater dosage to exert its effect. Also, the oral administrative insulin has to be absorbed from the gastric system and then enters the body circulation which takes too long and fails to work immediately. If the patient does not take the medicine on time, the consequence can be serious. Another type of study focuses on long lasting drug releasing carrier. To date, Sinha et al. disclosed (Sinha et al., "Chitosan microspheres as a potential carrier for drugs", International Journal of Pharmaceutics, 2004; 274(1-2):1-33) that chitosan can be a drug loading agent. There are some factors affecting drug release from the chitosan microspheres. These factors include molecular weight of chitosan, concentration of chitosan, drug content in the microspheres, physical state of the drug, density of cross-linking and additives. The releasing of insulin from the drug carrier is regulated by blood sugar concentration or pH value of the blood. For example, Anderson et al. disclosed in WO 2013/123492 A2 injectable insulin loaded microgels that are capable of modifying the amount of insulin released based on the patient's tissue glucose levels. When the blood sugar is too high, the structure of this type of drug carrier can be loosened or decomposed in order to release insulin. When the blood sugar level returns to normal, the drug carrier also returns to its former structure and suspend insulin releasing. This drug releasing mechanism is initialised by a specific blood sugar level and halts when the blood sugar level is low. The initialisation and halting cannot be easily controlled, causing wild fluctuation of blood sugar level. Under this mechanism, the dosage of insulin cannot be regulated whenever it is released, and a sudden bursting might occur along the time. The effective time window and storage level can hardly be managed.

Therefore, a long-lasting composition of drug carrier is of great interest and the abovementioned issues can be solved accompanied by higher effectiveness.

### SUMMARY

The instant disclosure provides a gel composition including a gel body, a chitosan decomposition enzyme and a drug. The gel body includes a first solution, a plurality of chitosan spheres and an alkaline chitosan stabilizing agent. The first solution contains water and oily solvent. The chitosan spheres are formed by chitosan self-assembly in the first solution. The alkaline chitosan stabilizing agent connects the chitosan spheres to form a gel body. The chitosan decomposition enzyme scatters in the gel body and is temperature sensitive. The drug scatters in the gel body.

According to an embodiment of the instant disclosure, the gel composition has a pH value ranging from 5 to 9.

According to an embodiment of the instant disclosure, the chitosan is an amphipathic chitosan.

According to an embodiment of the instant disclosure, the alkaline chitosan stabilizing agent is genipin, sodium β-glycerophosphate, NaHCO₃ or the combination thereof.

According to an embodiment of the instant disclosure, the chitosan decomposition enzyme is lysozyme, cellulase, chitinase or the combination thereof.

According to an embodiment of the instant disclosure, the drug scatters in and between the chitosan spheres.

According to an embodiment of the instant disclosure, the drug is insulin, insulin sensitizer, sulfonylurea or the combination thereof.

According to an embodiment of the instant disclosure, the gel composition further includes a diluent that adjusts a pH value of the gel composition. The diluent can be water or a mixture of water and an oily solvent, and the oily solvent is one selected from the group consisting of dimethyl sulfoxide (DMSO), ethanol, glycol and glycerol.

The instant disclosure also provides a method of manufacturing gel composition including preparing a chitosan solution having a concentration of 1 to 10 % (w/v) and solubilizing chitosan in a solvent. The chitosan self-assembles into a plurality of chitosan spheres in the solvent. Next, at a temperature of 4 to 10 degree Celsius, a drug is added to the chitosan solution to form a first solution. Subsequently, an alkaline chitosan stabilizing agent and a chitosan decomposition enzyme are added to and mixed in the first solution. The alkaline chitosan stabilizing agent connects the chitosan spheres to form the gel composition after resting and solidifying. A concentration of the chitosan stabilizing agent in the gel composition is 0.1 to 10% (w/v).

According to an embodiment of the instant disclosure, the chitosan is amphipathic chitosan.

According to an embodiment of the instant disclosure, the drug is insulin, insulin sensitizer, sulfonylurea or the combination thereof. A concentration of the drug in the gel composition is 0.1 to 10 mg/mL.

According to an embodiment of the instant disclosure, the method further includes adding a diluent to the chitosan solution to form the first solution and adjusting a pH value of the first solution to between 5 and 9. The diluent can be water or a mixture of water and an oily solvent, and the oily solvent is one selected from the group consisting of DMSO, ethanol, glycol and glycerol.

According to an embodiment of the instant disclosure, a concentration of the chitosan decomposition enzyme in the gel composition is 0.1 to 500 µg/mL.

The gel composition of the instant disclosure is a long-lasting, enzyme-induced, injectable drug releasing gel composition. By adding chitosan decomposition enzyme in the gel composition, the chitosan spheres decompose into segments, and the gel composition disassembles such that drug is released. By adjusting the enzyme concentration, the gel composition decomposition rate can be regulated. The drug releasing speed and amount can also be controlled in order to meet the effective dosage. The gel composition allows a more reliable, stable and sustainable treatment.

It is to be understood that both the foregoing general description and the following detailed description are by examples, and are intended to provide further explanation of the invention as claimed.

### BRIEF DESCRIPTION OF THE DRAWINGS

The invention can be more fully understood by reading the following detailed description of the embodiment, with reference made to the accompanying drawings as follows:
Fig. 1 is a cross-sectional view showing a gel composition in accordance with an embodiment of the instant disclosure;
Figs. 2A to 2C are schematic diagrams illustrating drug releasing from the gel composition in accordance with an embodiment of the instant disclosure;
Fig. 3 is a flow chart describing a method of manufacturing gel composition in accordance with an embodiment of the instant disclosure;
Fig. 4 is a graph showing weight percentage against time of a gel composition in accordance with an exemplary sample of the instant disclosure;
Fig. 5 is a graph showing an amount of released insulin against time of a gel composition in accordance with an exemplary sample of the instant disclosure; and
Fig. 6 is a graph showing blood sugar level of a diabetes-contracted mouse against time in accordance with an exemplary sample of the instant disclosure.

### DETAILED DESCRIPTION

Reference will now be made in detail to the present embodiments of the invention, examples of which are illustrated in the accompanying drawings. Wherever possible, the same reference numbers are used in the drawings and the description to refer to the same or like parts.

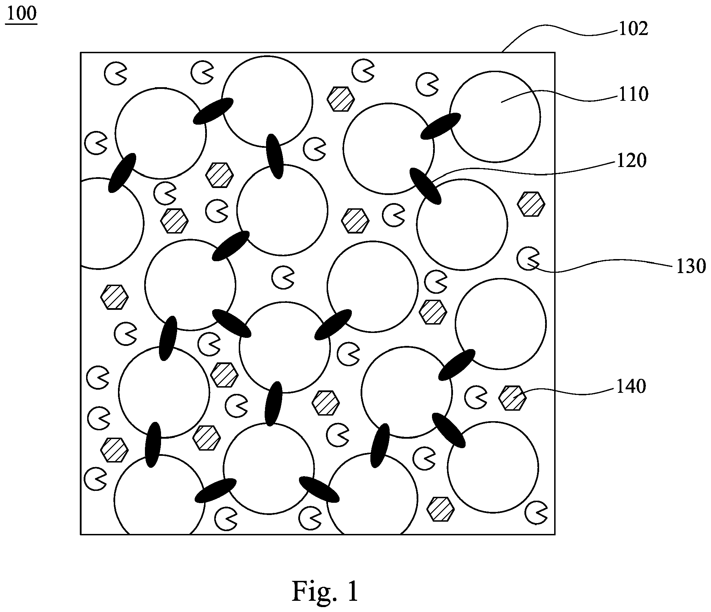

Please refer to Fig. 1 showing a cross-sectional view of a gel composition 100 in accordance with an embodiment of the instant disclosure. The gel composition 100 includes a plurality of chitosan spheres 110, an alkaline chitosan stabilizing agent 120, a chitosan decomposition enzyme 130 and a drug 140. The chitosan spheres 110 are formed by chitosan (not shown) self-assembly. The alkaline chitosan stabilizing agent 120 connects the chitosan spheres to form a gel body 102. The chitosan decomposition enzyme 130 scatters in the gel body 102 and decomposes the gel composition 100 at a temperature of 20 to 40 degree Celsius. The drug 140 is distributed in the gel body 102.

In one embodiment, a pH value of the gel composition is between 5 and 9. This pH range simulates the pH range in a body so as to avoid any adverse effect to the body.

In another embodiment, the chitosan is amphipathic chitosan. Amphipathic chitosan has hydrophilic end and hydrophobic end at the same time. Chitosan will self-assemble into chitosan sphere according to the hydrophilic or hydrophobic structural property.

The alkaline chitosan stabilizing agent 120 is used to stabilize the structure of the chitosan spheres 110. Alkaline chitosan stabilizing agent 120 is electronegativity, resulting in positively charged chitosan spheres 110 interconnected to form the gel body 102. In one embodiment, alkaline chitosan stabilizing agent can be genipin, sodium β-glycerophosphate, NaHCO₃ or the combination thereof.

Temperature can affect the activity and reaction rate of chitosan decomposition enzyme 130. Under standard condition and in appropriate temperature, enzyme activity increases along with the rise in temperature. The reaction rate increases as the temperature climbs. When the temperature exceeds an active temperature, the enzyme activity decreases sharply. When the temperature is below the active temperature, the enzyme does not exert its catalytic function but remain intact. Therefore, if the temperature rises again, the enzyme activity recovers gradually as well. An active temperature of chitosan decomposition enzyme 130 ranges between 20 and 40 degree Celsius. Under this temperature range, chitosan decomposition enzyme 130 can decompose chitosan. Therefore, when the gel composition 100 is not injected into a body, gel composition 100 has to be stored under low temperature (approximately 4 to 10 degree Celsius so as to prevent chitosan decomposition enzyme 130 from dismantling chitosan spheres 110 into segments. The integrity of the gel body 102 breaks down as the chitosan spheres 110 are segmented. In this way, the gel composition 100 also decomposes, and the drug 140 will be released. Accordingly, by adjusting the concentration of the chitosan decomposition enzyme 130, the rate of chitosan spheres 110 breakdown can also be regulated. In other words, the amount and period of drug 140 releasing can be manipulated.

In one embodiment, chitosan decomposition enzyme can be lysozyme, cellulase, chitinase or the combination thereof.

According to the dimension of the drug 140, it can be scatter inside of between the chitosan spheres 110. If the drug 140 is larger in size, it scatters in between the chitosan spheres 110. If the drug 140 is smaller in size, it is distributed inside the chitosan spheres 110. The gel composition 100 may contain more than one drug, and the drugs may be distributed inside and in between the chitosan spheres 110.

When the gel composition 100 is used to treat diabetes, the drug 140 can be insulin and/or other diabetes related medicine. Other associated drugs includes but not limited to insulin sensitizer and sulfonylurea. Insulin sensitizer can be, for example, Thiazolidinedione (TZD).

The gel composition may include diluent for adjusting pH value of the gel composition. The pH value of the gel composition should fall between 5 and 9. In one embodiment, the diluent can be water or a mixture of water and oily solvent. According to the embodiments, the oily solvent is one selected from the group consisting of but not limited to dimethyl sulfoxide (DMSO), ethanol, glycol and glycerol.

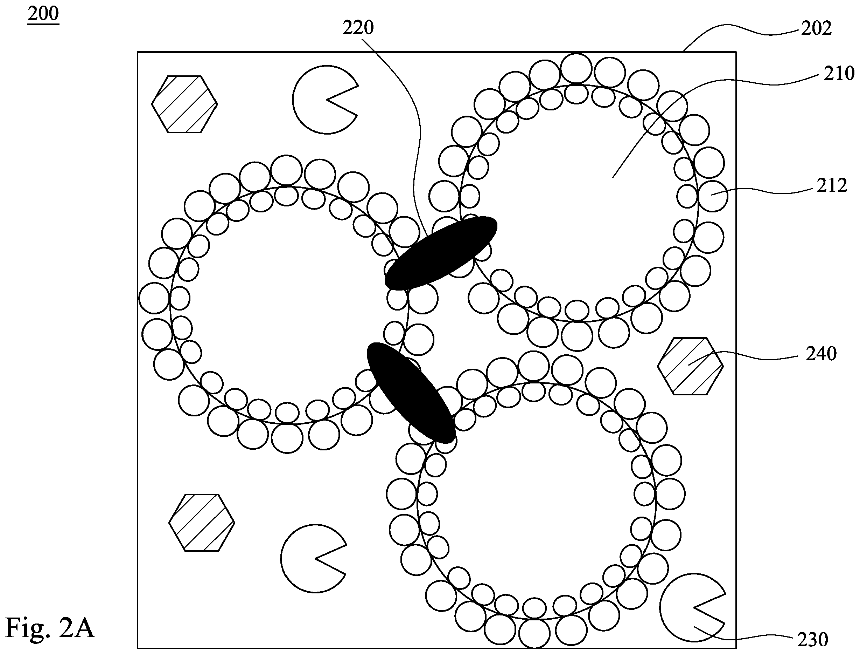

Please refer to Figs. 2A to 2C which are schematic diagrams illustrating drug releasing from the gel composition in accordance with an embodiment of the instant disclosure.

Please refer to Fig. 2A showing a diagram before gel composition 200 being injected to a body. The gel composition 200 includes a plurality of chitosan spheres 210, alkaline chitosan stabilizing agent 220, chitosan decomposition enzyme 230 and drug 240. The chitosan spheres 210 are formed by chitosan self-assembly. The alkaline chitosan stabilizing agent 220 connects the chitosan spheres to form a gel body 202. The chitosan decomposition enzyme 230 scatters in the gel body 202 and decomposes the gel composition 200 at a temperature of 20 to 40 degree Celsius. The drug 240 is distributed in the gel body 202. The temperature of the gel composition 200 is controlled below the active temperature of the chitosan decomposition enzyme 230 such that the chitosan decomposition enzyme 230 will not exert its catalytic function, and the gel composition 200 will not be decomposed.

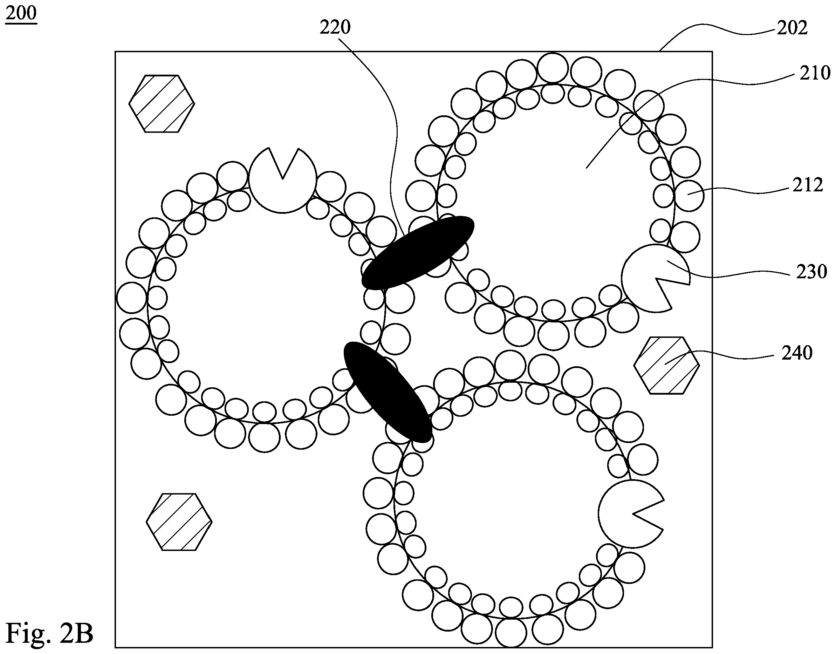

Please refer to Fig. 2B showing the gel composition 200 after being injected into a body. At this stage, because of body temperature, the temperature of the gel composition 200 arrives to the active temperature range of the chitosan decomposition enzyme 230. Subsequently, the chitosan decomposition enzyme 230 recovers from inactivity and decomposes the chitosan spheres 210.

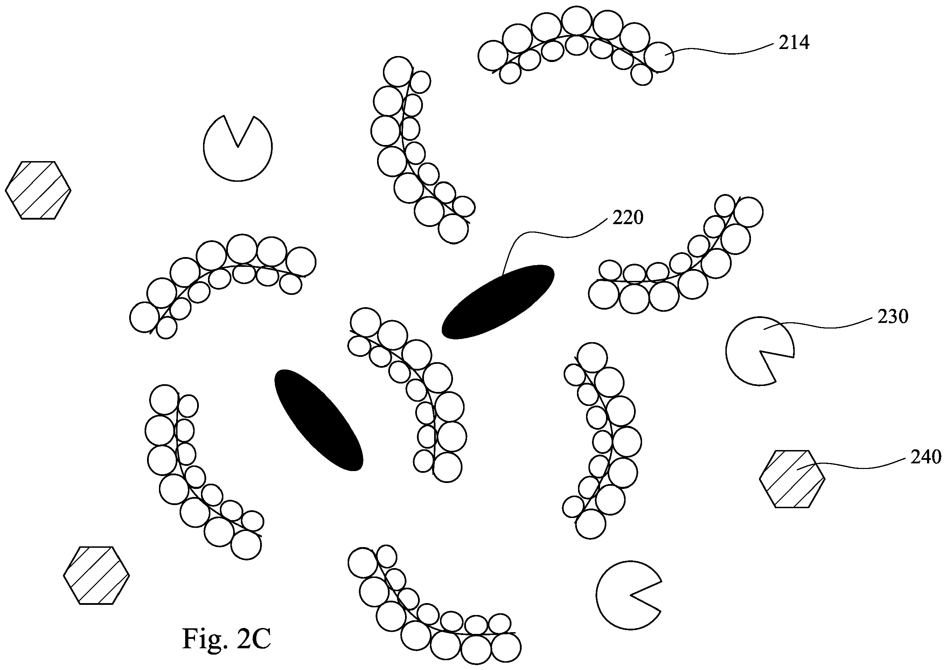

Please refer to Fig. 2C showing the gel composition 200 being decomposed. The chitosan decomposition enzyme 230 dismantles the chitosan spheres 210 into chunks of chitosan segments 214. The chitosan spheres 210 lose its integrity to chitosan segments 214, and the structure of gel body 202 breaks down. Therefore, as the gel composition 200 breaks down, the drug 240 is released and transmitted to the target site for treatment.

The gel composition of the instant disclosure can be used to carry drugs. By adding the chitosan decomposition enzyme, the drug can be released in a stable and continuous fashion. In addition, by adjusting the concentration of chitosan decomposition enzyme and the drug in the gel composition, the amount and period of drug released can be regulated.

### Method of manufacturing the gel composition

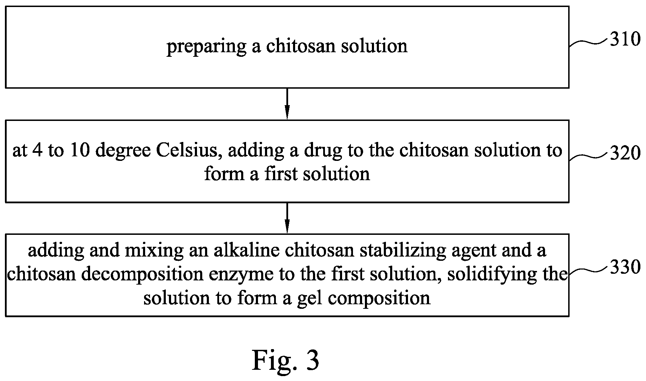

Please refer to Fig. 3 showing a flow chart of a method of manufacturing the gel composition. Firstly, chitosan solution 310 has to be prepared. Next, at 4 to 10 degree Celsius, the drug is added to the chitosan solution 310 to form a first solution 320. Then, the alkaline chitosan stabilizing agent and the chitosan decomposition enzyme are added to the first solution and mixed. After solidification, the gel composition 330 is formed.

In step 310, chitosan solution is prepared, and the solution contains chitosan in the solvent. Chitosan self-assembles into a plurality of chitosan spheres scattering in the solvent. The chitosan concentration is 1 to 10% (w/v), and the solvent might be water or a mixture of water and ethanol.

Weight/volume percentage (w/v) refers to the weight of solute in gram in 100 ml solution. For example, 1 g solute in 100 ml solution has a concentration of 1% (w/v).

In one embodiment, chitosan is amphipathic chitosan. More specifically, it has hydrophilic and hydrophobic property at the same time. When preparing the chitosan solution, chitosan self-assembles according to hydrophilic or hydrophobic to form the chitosan spheres.

In one embodiment, the drug is insulin, insulin sensitizer, sulfonylurea or the combination thereof. The concentration of the drug in the gel composition falls between 0.1 and 10 mg/mL.

In one embodiment, in step 320 further includes adding a diluent to the chitosan solution to form a first solution, and the pH value of the first solution can be adjusted to 5 to 9 in the addition of the diluent. It is to simulate a condition similar to the pH value in the body. The diluent can be water or a mixture of water and oily solvent. The concentration of the oily solvent in the mixture falls between 1 and 20%. The oily solvent includes but not limited to dimethyl sulfoxide, ethanol, glycol and glycerol.

The negatively charged alkaline chitosan stabilizing agent facilitates the connection between the positively charged chitosan spheres to form a stable gel structure. Therefore, in step 330, after the addition and mixing of alkaline chitosan stabilizing agent into the first solution, the alkaline chitosan stabilizing agent connects the chitosan spheres. After resting, the solution solidifies, chitosan decomposition enzyme and drug in the gel body together form the gel composition. The concentration of alkaline chitosan stabilizing agent in the gel composition ranges between 0.1 and 10% (w/v), and chitosan decomposition enzyme breaks down the gel composition at a temperature of 20 to 40 degree Celsius.

The concentration of chitosan decomposition enzyme can be adjusted according to the required amount and period of drug releasing. The higher the concentration, the faster the decomposition rate of the gel composition. In this way, the amount of the drug releasing is more in a shorter time period. In one embodiment, the concentration of chitosan decomposition enzyme in the gel composition ranges from 0.1 to 500 µg/mL.

The gel composition of the instant disclosure is an injectable, long-lasting, enzyme-induced drug releasing gel composition. The addition of chitosan decomposition and drug in the gel composition can regulate drug releasing by the decomposition rate of the gel composition. Furthermore, by adjusting different concentrations of chitosan decomposition enzyme and the drug, the amount and time period of drug releasing can be fine tuned.

A number of examples are provided herein to elaborate the gel composition of the instant disclosure. However, the examples are for demonstration purpose alone, and the instant disclosure is not limited thereto.

### Example 1

Example 1 used two different concentrations of chitosan, namely 2.4% (w/v) and 3% (w/v). Two different concentrations of chitosan decomposition enzyme, which were 10 and 100 µg/mL, were added to form different gel composition. A control sample without any chitosan decomposition enzyme was used for the weight comparison among different gel composition. In this experiment, the alkaline chitosan stabilizing agent was sodium β-glycerophosphate, and the chitosan decomposition enzyme was lysozyme. It should be noted that once the gel composition is injected inside a body, because of the flow of body fluid, the decomposed chitosan segments will be taken away from the injection spot. Therefore, this experiment simulated the condition in a body, and the gel composition was deposited in a buffer solution that was close to body fluid. The buffer solution was renewed regularly, and the chitosan segments are removed therefrom. The weight change of the gel composition was then recorded.

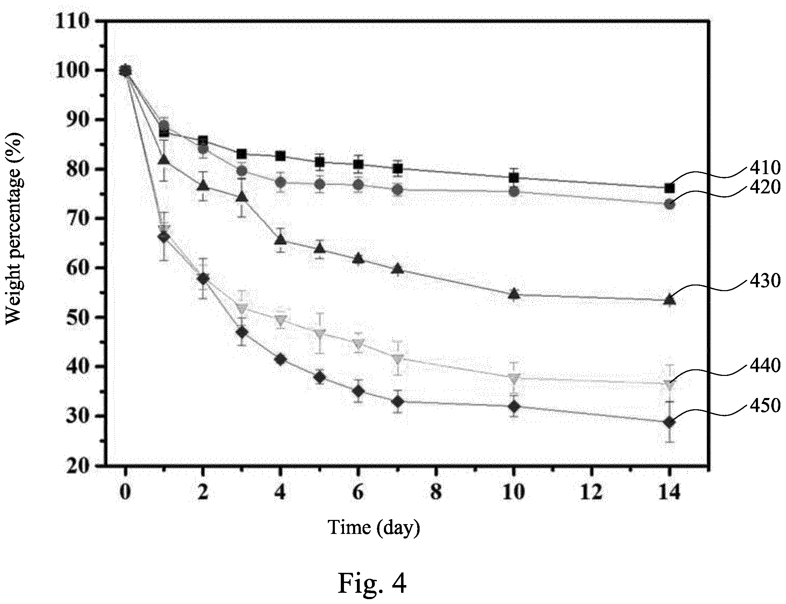

Please refer to Fig. 4 showing a graph of weight percentage of the gel composition against time. The weight on day 0 was 100% when the gel composition was not added, and the remaining weight percentage was in relation to day 0. Lines 410, 420, 430, 440 and 450 represent gel composition with no lysozyme and 3% chitosan, 10 µg/mL lysozyme and 3% chitosan, no lysozyme and 2.4% chitosan, 10 µg/mL lysozyme and 2.4% chitosan and 100 µg/mL lysozyme and 2.4% chitosan respectively. The weight change along the time course is shown in Fig. 4. The gel composition with higher chitosan concentration had slower decomposition speed. At day 14, the difference between lines 410 and 430 was significant which was more than 20%. Moreover, as the concentration of lysozyme increased, higher decomposition speed was observed in 2.4% and 3% gel composition. After fortnight, line 450 lost almost 70% of its weight. As a result, by adjusting the concentration of gel body and enzyme, the decomposition timing of the gel composition can be controlled. The concentration formula can be tuned according to requirement such that the duration of the gel composition and decomposition rate can be controlled.

### Example 2

Example 2 used different concentrations of chitosan solution and chitosan decomposition enzyme in the gel composition. The accumulative drug releasing amount was measured. In this experiment, the alkaline chitosan stabilizing agent was sodium β-glycerophosphate, the chitosan decomposition enzyme was lysozyme, and drug was insulin with a concentration of 5 mg/mL.

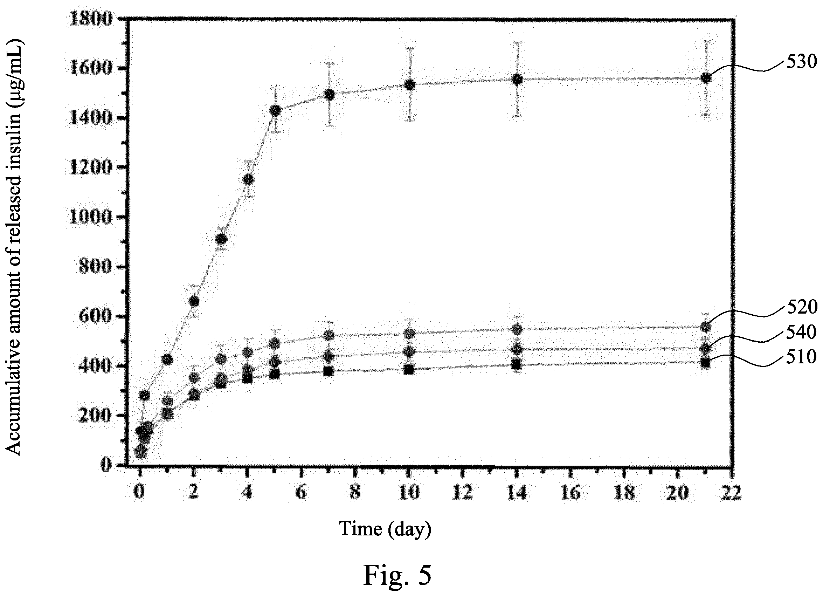

Please refer to Fig. 5 showing the accumulative releasing amount of insulin from the gel composition against time. Lines 510, 520, 530, 540 represent gel composition with no lysozyme and 2.4% chitosan, 10 µg/mL lysozyme and 2.4% chitosan, 100 µg/mL lysozyme and 2.4% chitosan, and 10 µg/mL lysozyme and 3% respectively. According to Fig. 5, it can be seen that as the lysozyme concentration increased, the amount of released insulin greatly increased as well. Compared to gel composition without lysozyme (line 510), the gel composition with 10 µg/mL lysozyme (line 520) had an almost doubled insulin releasing amount. When the lysozyme concentration went up to 100 µg/mL (line 530), the amount of released insulin increased five folds. Therefore, the amount of insulin released into the body can be adjusted according to different requirement so as to control the blood sugar level.

### Example 3

Example 3 was conducted *in vivo* on diabetes contracted mice. The efficacy of gel composition was studied in this experiment. In this experiment, the alkaline chitosan stabilizing agent was sodium β-glycerophosphate, the chitosan decomposition enzyme was lysozyme, and the drug was insulin with a concentration of 5 mg/mL.

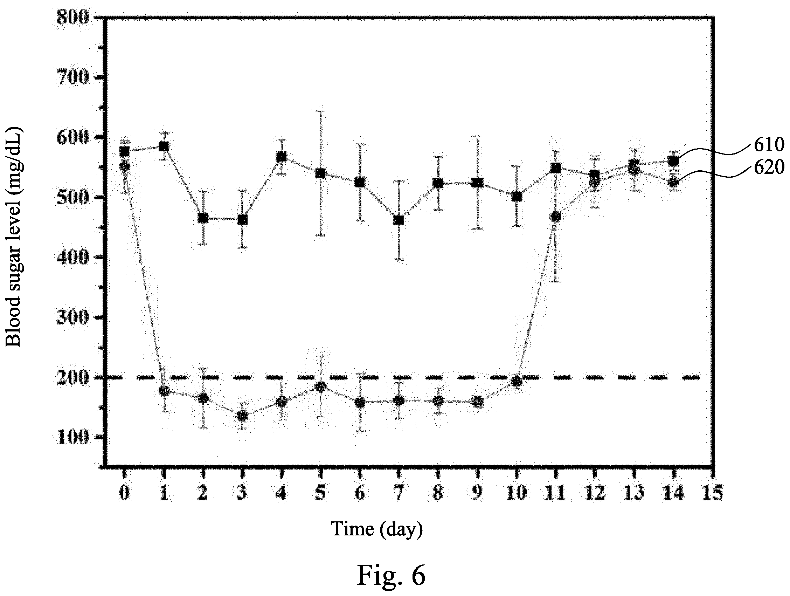

Please refer to Fig. 6 showing the blood sugar level of the mice against time. Lines 610 and 620 respectively represent a control group with no gel composition injection and an example with gel composition injection. The blood sugar level change can be seen along the time course. The gel composition in this experiment had 10 µg/mL lysozyme and 2.4% chitosan. Normal blood sugar level should be controlled below 200 mg/dl. In Fig. 6, mice without gel composition injection (line 610) had a blood sugar level higher than 400 mg/dl all the time which was far beyond normal blood sugar level. On the other hand, mice with the injection of 10 µg/mL lysozyme and 2.4% chitosan gel composition (line 620) had a blood sugar level lower than 200 mg/dl through the time which was in a normal range. The efficacy lasted for 10 days. Therefore, the gel composition of the instant disclosure can release insulin in a stable and continuous mode. The gel composition helps to control the blood sugar level in longer term. One injection can last 10 days, and the metal and physical burden of drug administration is greatly reduced.

After injection of the gel composition of the instant disclosure, it is unlikely to induce immune response because of its high biocompatibility.

The gel composition of the instant disclosure is an injectable, long-lasting, enzyme induced drug releasing gel composition. By adding chitosan decomposition enzyme to the gel composition, the chitosan spheres are broken down into segments and the drug will be released therefrom. Although other research also suggests that the gel can be decomposed by enzymes in the body, the enzyme concentration and decomposition rate cannot be well regulated. The gel composition of the instant disclosure contains chitosan decomposition enzyme such that the gel decomposition and drug releasing can be controlled down to its rate, amount and time period. The required dosage in the treatment can be easily achieved, and the efficacy lasts longer in a stable trend. In addition, in one embodiment, the gel composition can be injected once every two weeks so as to reduce the number of injection. The stable releasing from the gel composition allows accurate measurement of drug concentration in the body and the remaining storage. The blood sugar level is better regulated under this condition. In short, the gel composition of the instant disclosure reduces the number of injection and brings quality life to the patients.

## Claims

1. A gel composition, comprising:
a gel body (102) including:
- a first solution containing water and oily solvent;
- a plurality of chitosan spheres (110), each of the chitosan spheres (110) being self-assembled by chitosan in the first solution;
- an alkaline chitosan stabilizing agent (120) connecting the chitosan spheres (110);
a chitosan decomposition enzyme (130) scattering in the gel body (102), wherein the chitosan decomposition enzyme is temperature sensitive; and
a drug (140) scattering in the gel body (102).

2. The gel composition of claim 1, wherein the gel composition has a pH value ranging from 5 to 9.

3. The gel composition of claim 1, wherein the chitosan is an amphipathic chitosan.

4. The gel composition of claim 1, wherein the alkaline chitosan stabilizing agent (120) is genipin, sodium β-glycerophosphate, NaHCO₃ or a combination thereof.

5. The gel composition of claim 1, wherein the chitosan decomposition enzyme (130) is lysozyme, cellulase, chitinase or a combination thereof.

6. The gel composition of claim 1, wherein the drug (140) scatters in and between the chitosan spheres (110).

7. The gel composition of claim 1, wherein the drug (140) is insulin, insulin sensitizer, sulfonylurea or a combination thereof.

8. The gel composition of claim 1, further comprising:
a diluent that adjusts a pH value of the gel composition, wherein the diluent is water or a mixture of water and an oily solvent, and the oily solvent is dimethyl sulfoxide, ethanol, glycol or glycerol.

9. A method of manufacturing gel composition, comprising:
preparing a chitosan solution having a concentration of 1 to 10 % (w/v), solubilizing chitosan in a solvent, the chitosan self-assembling into a plurality of chitosan spheres in the solvent;
at a temperature of 4 to 10 degree Celsius, adding a drug to the chitosan solution to form a first solution; and
adding and mixing an alkaline chitosan stabilizing agent and a chitosan decomposition enzyme to the first solution, the alkaline chitosan stabilizing agent connecting the chitosan spheres, solidifying the first solution to form the gel composition, wherein a concentration of the chitosan stabilizing agent in the gel composition is 0.1 to 10% (w/v).

10. The method of claim 9, wherein the chitosan is amphipathic chitosan.

11. The method of claim 9, wherein the drug is insulin, insulin sensitizer, sulfonylurea or a combination thereof.

12. The method of claim 11, wherein a concentration of the drug in the gel composition is 0.1 to 10 mg/mL.

13. The method of claim 9, further comprising:
adding a diluent to the chitosan solution to form the first solution; and
adjusting a pH value of the first solution to between 5 and 9.

14. The method of claim 13, wherein the diluent is water or a mixture of water and an oily solvent, and the oily solvent is dimethyl sulfoxide, ethanol, glycol or glycerol.

15. The method of claim 9, wherein a concentration of the chitosan decomposition enzyme in the gel composition is 0.1 to 500 µg/mL.

## Patentansprüche

1. Gelzusammensetzung, umfassend:
einen Gelkörper (102), welcher enthält:
- eine erste Lösung, die Wasser und ein öliges Lösungsmittel enthält;
- eine Vielzahl von Chitosan-Kugeln (110), wobei jede der Chitosan-Kugeln (110) durch Chitosan selbst zusammengefügt ist;
- ein alkalisches Chitosan-Stabilisierungsmittel (120), das die Chitosan-Kugeln (110) verbindet;
ein Chitosan-Abbauenzym (130), das in dem Gelkörper (102) verteilt ist, wobei das Chitosan-Abbauenzym temperaturempfindlich ist; und
ein Arzneimittel (140), das in dem Gelkörper (102) verteilt ist.

2. Gelzusammensetzung nach Anspruch 1, wobei die Gelzusammensetzung einen pH-Wert im Bereich von 5 bis 9 aufweist.

3. Gelzusammensetzung nach Anspruch 1, wobei das Chitosan ein amphipathisches Chitosan ist.

4. Gelzusammensetzung nach Anspruch 1, wobei das alkalische Chitosan-Stabilisierungsmittel (120) Genipin, Natrium-β-glycerophosphat, NaHCO₃ oder eine Kombination davon ist.

5. Gelzusammensetzung nach Anspruch 1, wobei das Chitosan-Abbauenzym (130) Lysozym, Cellulase, Chitinase oder eine Kombination davon ist.

6. Gelzusammensetzung nach Anspruch 1, wobei das Arzneimittel (140) in und zwischen den Chitosan-Kugeln (110) verteilt ist.

7. Gelzusammensetzung nach Anspruch 1, wobei das Arzneimittel (140) Insulin, Insulinsensibilisator, Sulfonylharnstoff oder eine Kombination davon ist.

8. Gelzusammensetzung nach Anspruch 1, zudem umfassend:
ein Verdünnungsmittel, das einen pH-Wert der Gelzusammensetzung einstellt, wobei das Verdünnungsmittel Wasser oder ein Gemisch aus Wasser und einem öligen Lösungsmittel ist und das ölige Lösungsmittel Dimethylsulfoxid, Ethanol, Glykol oder Glycerin ist.

9. Verfahren zur Herstellung einer Gelzusammensetzung, umfassend:
Herstellen einer Chitosanlösung mit einer Konzentration von 1 bis 10% (Gew./Vol.),
Solubilisieren von Chitosan in einem Lösungsmittel, wobei sich das Chitosan zu einer Vielzahl von Chitosan-Kugeln in dem Lösungsmittel selbst zusammenfügt;
bei einer Temperatur von 4 bis 10°C Hinzufügen eines Arzneimittels zur Chitosanlösung, so dass eine erste Lösung gebildet wird; und
Zugeben und Mischen eines alkalischen Chitosan-Stabilisierungsmittels und eines Chitosan-Abbauenzyms zu der ersten Lösung, wobei das alkalische Chitosan-Stabilisierungsmittel die Chitosan-Kugeln verbindet, Verfestigen der ersten Lösung, so dass die Gelzusammensetzung gebildet wird, wobei eine Konzentration des Chitosan-Stabilisierungsmittels in der Gelzusammensetzung 0,1 bis 10% (Gew./Vol.) beträgt.

10. Verfahren nach Anspruch 9, wobei das Chitosan amphipathisches Chitosan ist.

11. Verfahren nach Anspruch 9, wobei das Arzneimittel Insulin, Insulinsensibilisator, Sulfonylharnstoff oder eine Kombination davon ist.

12. Verfahren nach Anspruch 11, wobei eine Konzentration des Arzneimittels in der Gelzusammensetzung 0,1 bis 10 mg/ml beträgt.

13. Verfahren nach Anspruch 9, zudem umfassend:
Hinzufügen eines Verdünnungsmittels zu der Chitosanlösung, so dass die erste Lösung gebildet wird; und
Einstellen eines pH-Wertes der ersten Lösung auf einen Wert zwischen 5 und 9.

14. Verfahren nach Anspruch 13, wobei das Verdünnungsmittel Wasser oder ein Gemisch aus Wasser und einem öligen Lösungsmittel ist und das ölige Lösungsmittel Dimethylsulfoxid, Ethanol, Glykol oder Glycerin ist.

15. Verfahren nach Anspruch 9, wobei eine Konzentration des Chitosan-Abbau-Enzyms in der Gelzusammensetzung 0,1 bis 500 pg/ml beträgt.

## Revendications

1. Composition de gel, comprenant :
un corps de gel (102) comprenant :
- une première solution contenant de l'eau et un solvant huileux ;
- une pluralité de sphères de chitosane (110), chacune des sphères de chitosane (110) étant autoassemblée par le chitosane dans la première solution ;
- un agent de stabilisation de chitosane alcalin (120) reliant les sphères de chitosane (110) ;
- une enzyme de décomposition de chitosane (130) diffusant dans le corps de gel (102), l'enzyme de décomposition de chitosane étant sensible à la température ; et
- un médicament (140) diffusant dans le corps de gel (102).

2. Composition de gel selon la revendication 1, la composition de gel ayant une valeur de pH dans la plage de 5 à 9.

3. Composition de gel selon la revendication 1, dans laquelle le chitosane est un chitosane amphipathique.

4. Composition de gel selon la revendication 1, dans laquelle l'agent de stabilisation de chitosane alcalin (120) est la génipine, le β-glycérophosphate de sodium, NaHCO₃ ou une combinaison de ceux-ci.

5. Composition de gel selon la revendication 1, dans laquelle l'enzyme de décomposition de chitosane (130) est un lysozyme, une cellulase, une chitinase ou une combinaison de ceux-ci.

6. Composition de gel selon la revendication 1, dans laquelle le médicament (140) diffuse dans et entre les sphères de chitosane (110).

7. Composition de gel selon la revendication 1, dans laquelle le médicament (140) est l'insuline, un insulinosensibilisateur, une sulfonylurée ou une combinaison de ceux-ci.

8. Composition de gel selon la revendication 1, comprenant en outre :
un diluant qui ajuste une valeur de pH de la composition de gel, dans laquelle le diluant est l'eau ou un mélange d'eau et d'un solvant huileux, et le solvant huileux est le diméthylsulfoxyde, l'éthanol, le glycol ou le glycérol.

9. Procédé de fabrication d'une composition de gel, comprenant :
la préparation d'une solution de chitosane ayant une concentration de 1 à 10 % (m/v), la solubilisation du chitosane dans un solvant, l'autoassemblage du chitosane en une pluralité de sphères de chitosane dans le solvant ;
à une température de 4 à 10 degrés Celsius, l'ajout d'un médicament à la solution de chitosane pour former une première solution ; et
l'ajout et le mélange d'un agent de stabilisation de chitosane alcalin et d'une enzyme de décomposition de chitosane avec la première solution, l'agent de stabilisation de chitosane alcalin reliant les sphères de chitosane, la solidification de la première solution pour former la composition de gel, dans lequel la concentration de l'agent de stabilisation de chitosane dans la composition de gel est de 0,1 à 10 % (m/v).

10. Procédé selon la revendication 9, dans lequel le chitosane est un chitosane amphipathique.

11. Procédé selon la revendication 9, dans lequel le médicament est l'insuline, un insulinosensibilisateur, une sulfonylurée ou une combinaison de ceux-ci.

12. Procédé selon la revendication 11, dans lequel la concentration du médicament dans la composition de gel est de 0,1 à 10 mg/ml.

13. Procédé selon la revendication 9, comprenant en outre :
l'ajout d'un diluant à la solution de chitosane pour former la première solution ; et l'ajustement d'une valeur de pH de la première solution entre 5 et 9.

14. Procédé selon la revendication 13, dans lequel le diluant est l'eau ou un mélange d'eau et d'un solvant huileux, et le solvant huileux est le diméthylsulfoxyde, l'éthanol, le glycol ou le glycérol.

15. Procédé selon la revendication 9, dans lequel la concentration de l'enzyme de décomposition de chitosane dans la composition de gel est de 0,1 à 500 pg/ml.
